# EUROPEAN PATENT APPLICATION

(11) **EP 3 832 665 A1**
(43) Date of publication of application: **09.06.2021**
(21) Application number: 19212800.7
(22) Date of filing: 02.12.2019
(51) Int. Cl.: G16H 50/70, G16H 70/00, G06F 16/36, G06F 40/30

(54) **GENERATING OR MODIFYING AN ONTOLOGY REPRESENTING RELATIONSHIPS WITHIN INPUT DATA**

(71) Applicant: Koninklijke Philips N.V., 5656 AG Eindhoven (NL)
(72) Inventor: HU, Yiyi, 5656 AE Eindhoven (NL); LI, Yan, 5656 AE Eindhoven (NL); SUN, Huichuan, 5656 AE Eindhoven (NL); ZHOU, Jian, 5656 AE Eindhoven (NL); FAN, Jia, 5656 AE Eindhoven (NL)
(74) Representative: Philips Intellectual Property & Standards

(57) **Abstract**

A method and system for orchestrating the analysis of input data containing data items, e.g. medical text, with at least two processing techniques. Two different processing techniques process a respective portion of the input data to identify relationships between the data items of the input data. The accuracy of each processing technique is then determined, and the size of the respective portions is automatically changed for a subsequent iteration of processing input data based the determined accuracy.

## Description

### FIELD OF THE INVENTION

The present invention relates to the field of generating/modifying an ontology, which represents relationships within input data.

### BACKGROUND OF THE INVENTION

Medical literature databases, such as PubMed, are rich data sources for clinical knowledge mining. These databases provide a wealth of information that can be used to establish clinical/treatment guidelines, e.g. by a researcher identifying relationships between treatments and success levels indicated in the medical literature.

However, it is extremely labor-intensive for human researchers to read though the literature to establish guidelines. There is therefore a demand for an intelligent processing system that is able to detect modalities discussed by papers, summarize the study outcomes automatically and present to clinicians.

A first step in the design of such a system is to create an ontology that represents relationships between different data items (e.g. words or phrases) of the medical literature. This ontology could also serve as a modality detector. An ontology may, for example, encode relations using triple stores or a graph structure/database (e.g. which employs a property graph model). However, constructing an ontology is extremely challenging, e.g. due to complex combinatory modalities contained in free-text, complex dependencies and so on.

Existing solutions to creating an ontology, or modifying an existing ontology, employ machine-learning algorithms for processing the medical literature. However, the creation of training data for such machine-learning algorithms is difficult and time consuming, requiring a large number of participants to provide enough training data for accurate processing of the medical literature.

There is therefore a desire to provide an improved method of generating (or modifying) an ontology that can be implemented on a computer. In particular, there is a desire to facilitate the accurate implementation of automatic determination of relationships between input data on a computer.

### SUMMARY OF THE INVENTION

The invention is defined by the claims.

According to examples in accordance with an aspect of the invention, there is provided a computer-implemented method of generating or modifying an ontology representing relationships within input data. The method comprises iteratively: obtaining input data for processing, the input data comprising a plurality of data items; processing a first portion of the input data using a first processing technique configured to identify relationships between different data items of the input data, the size of the first portion being a first percentage of the input data; processing a second, different portion of the input data using a second, different processing technique configured to identify relationships between different data items of the input data, the size of the second portion being a second percentage of the input data; generating or modifying an ontology based on the relationships between the different data items identified by the first and second processing techniques; determining an accuracy of each of the first and second processing techniques; and adjusting a size of the first and second percentages, for processing of future input data, based on the determined accuracy of each of the first and second processing techniques.

The proposed invention provides an automated or semi-automated methodology for generating an ontology, such as a knowledge graph or knowledge base, which identifies relationships between different data items or entities of input data. The present invention thereby solves a problem of how to accurately implemented (semi)automated determination of such relationships using a computer or processor.

Use of (at least) two different processing techniques allows the most accurate technique to be used for generating the ontology. By iteratively adjusting a percentage of each proportion of input data, the most accurate technique can be weighted to provide a greater influence on the generated/modified ontology. This improves an accuracy of the ontology, and avoids bottlenecking of any single processing technique.

The proposed approach controls how processing techniques are utilized in order to generate an ontology. In particular, the proposed invention facilitates the use of (at least) two processing techniques by a computer to process input data, thus facilitating the accurate implementation of an automatic determination of relationships within input data on a computer.

Moreover, by accurately identifying relationships, the generated ontology can be used for more accurate processing of the input data, e.g. for later searching tools. By way of example, if the input data is textual medical data, then a medical question and answering system may retrieve appropriate answers to a question more accurately (as a relationship between a query and an answer may be established by the ontology). Ontology based searching is simpler and more effective than other

Preferably, the ontology identifies at least two data items of the input data and the relationship between the two data items. Suitable examples of an ontology include a triplestore, which stores one or more (semantic) triples, a knowledge graph/base, an entity relationship model and so on.

In some embodiments, the first and/or second processing technique each employ a plurality of subordinate processing techniques to identify relationships between different data items of the input data. Thus, the first processing technique may effectively be a first set of one or more processing techniques, and the second processing technique be a second, different set of one or more processing techniques.

In some embodiments, the accuracy may be determined based on user inputs (e.g. correcting a generated ontology), i.e. effectively a manual correction. In other embodiments, the accuracy may be determined using a ground-truth data base to determine an accuracy of each processing technique. Suitable examples of such methods will be later described.

Preferably, the input data for processing comprises textual data, each data item representing at least one word of the textual data. Textual data is particularly appropriate for use with the present invention, as textual data indicates relationships (e.g. semantic relationships or parent-child, i.e. hypernym or hyponym, relationships) that are typically difficult to identify using automated methods. The proposed invention provides a (semi)automated method of processing potentially difficult textual data that can be implemented on a computer with greater accuracy.

In particular embodiments, the textual data may comprise medical literature, e.g. one or more articles focusing upon a target disease domain (such as HCC) or review articles about the effectiveness of treatments for a certain disease. The present invention is particularly advantageous for use in medical literature, as medical literature often defines potentially complex relationships that are difficult for automated methods to intuitively identify.

The step of obtaining input data may comprise: obtaining initial input data comprising textual data; processing the initial input data using a natural language processing technique to detect entities within the initial input data; and normalizing the detected entities into standardized encoding, to thereby generate the input data for further processing.

The first processing technique may comprise a rule-based processing technique and the. The second processing technique may comprise a machine-learning processing technique, such as a neural network processing technique.

Machine-learning processing techniques typically need a large body of training data in order to be highly accurate. However, once trained, they tend to provide more accurate results than a mere rule-based learning processing technique.

The inventors have recognized that when initially processing input data, a machine-learning method may not be sufficiently trained to be highly accurate. As more and more input data is processed (e.g. and corrected by a user) or more training data becomes available, the machine-learning processing technique may be retrained to gradually become more accurate over time. For example, user-corrected ontologies generated by the method may be used as additional training data for first and/or second processing techniques. Thus, the proposed invention is particularly advantageous when used to modify a percentage of input data processed via a rules-based technique as via a machine-learning processing technique.

The rules-based technique may, in particular embodiments, be a (language) pattern matching technique adapted to identify semantic or grammatical patterns within textual data to thereby derive the relationship between different data items.

The machine-learning processing technique may be any suitable learning-based technique capable of identifying entities and/or relationships within input data. Examples include a support-vector machine and neural network processing techniques.

Where the first/second processing technique employs a plurality of subordinate processing techniques, each subordinate processing technique may be a rule-based processing technique or a machine-learning processing technique respectively.

In at least one embodiment, for the first iteration, the size of the first percentage is greater than the size of the second percentage. This embodiment takes account of the understanding that the rule-based processing technique will likely be initially more accurate than the machine-learning processing technique. Thus, it would be preferable to, at least initially, rely on the rule-based processing technique more than the machine-learning processing technique.

In further embodiments, for the first iteration, the size of the first percentage is between 80% and 95% and the size of the second percentage is between 5% and 20%, wherein the total of the first and second percentages is no greater than 100%.

The step of determining an accuracy of each of the first and second processing techniques may comprise: obtaining validation input data comprising a plurality of validation data items; obtaining validation answer data indicating relationships between the validation data items of the validation input data; processing validation input data using the first processing technique to generate first validation output data predicting relationships between different validation data items of the validation input data; comparing the first validation output data to the validation answer data to determine an accuracy of the first processing technique; processing the validation input data using the second processing technique to generate second validation output data predicting relationships between different validation data items of the validation input data; and comparing the second validation output data to the validation answer data to determine an accuracy of the second processing technique.

Methods of determining an accuracy using validation information (i.e. validation input and answer data) will be well known to the skilled person, and may include determining a sensitivity, specificity and/or error function of a processing technique using the validation information (any of which may act as a measure of accuracy for the purpose of the present invention).

The step of determining an accuracy of each of the first and second processing techniques may comprise: receiving one or more user correction signals, each user correction signal indicating a user-identified correction or change to a relationship between the different data items identified by the first and second processing techniques; and determining an accuracy of the first and second processing techniques based on the user correction signals. Thus, user corrections may be used to determine an accuracy of the first and second processing technique. This makes the method semi-automated.

The method may further comprise, between iterations, retraining or further training at least one of the first and second processing techniques using training input data and training answer data. In some embodiments, a user-corrected ontology (initially generated by a method, and subsequently corrected) may be used to (re)train the first and/or second processing technique. This improves an efficiency of training the first/second processing techniques, by more efficiently obtaining suitable training data. In such an embodiment, the user-corrected ontology may act as the training answer data and the associated input data may act as the training input data. Of course, the (re)training may be performed after a predetermined number of iterations has been performed or after a predetermined time period of performing the iterative steps of the method (e.g. 5 hours) has elapsed.

The ontology is preferably a tree-based structure comprising nodes, each node representing a different data item, and connections between nodes, each connection representing a relationship between data items represented by the nodes. For example, the ontology may be a graph structure storable in a graph database (e.g. which employs a property graph modelling approach).

The step of adjusting a size of the first and second percentages may comprise: determining whether a manual override signal provides information on a user's desired size of the first and/or second percentage; and in response to the manual override signal providing information on a user's desired size, adjusting the size of the first and/or second percentage based on the manual override signal. Thus, the first/second percentage may be controlled by a user via a manual override signal.

The step of generating or modifying an ontology may further comprise: determining whether a user input signal provides information on relationships between different data items of the input data; and in response to the user input signal providing information on relationships, generating or modifying the ontology further based on the user input signal.

The method may further comprise processing a third portion (different to the first and second portions) of the input data using a third, different processing technique configured to identify relationships between different data items of the input data, the size of the third portion being a third percentage of the input data. The step of generating or modifying an ontology may be further based on the relationships identified by the third processing technique. The step of determining an accuracy may further comprise determining an accuracy of the third processing technique and the step of adjusting a size may further comprise adjusting a size of the third percentage based on the determined accuracy of at least the third processing technique.

Thus, the methodology may effectively be expanded to operate with three or more processing techniques, through appropriate adaptations to the procedure. The third processing technique may integrate both rule-based and learning based processing techniques, i.e. be a hybrid processing technique.

There is also proposed a computer program comprising code means for implementing any previously described method when said program is run on a processing system.

There is also proposed a processing system for generating or modifying an ontology representing relationships within input data. The processing system comprises: an input module adapted to obtain input data for processing, the input data comprising a plurality of data items; a first processing module adapted to process a first portion of the input data using a first processing technique configured to identify relationships between different data items of the input data, the size of the first portion being a first percentage of the input data; a second processing module adapted to process a second, different portion of the input data using a second, different processing technique configured to identify relationships between different data items of the input data, the size of the second portion being a second percentage of the input data; an ontology generating module adapted to generate or modify an ontology based on the relationships between the different data items identified by the first and second processing techniques; an accuracy determining module adapted to determine an accuracy of each of the first and second processing techniques; and a size adjustment module adapted to adjust a size of the first and second percentages, for processing of future input data, based on the determined accuracy of each of the first and second processing techniques.

The elements of the processing system may be appropriately adapted for carrying out steps of any previously described method, or additional elements may be added for carrying out one or more such steps.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates the architecture of a processing system according to an embodiment;
Fig. 2 conceptually illustrates an ontology generated by an embodiment; and
Fig. 3 is a flow chart illustrating a method according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

According to a concept of the invention, there is proposed a method and system for orchestrating the analysis of input data containing data items, e.g. medical text, with at least two processing techniques. Two different processing techniques process a respective portion of the input data to identify relationships between the data items of the input data. The accuracy of each processing technique is then determined, and the size of the respective portions is automatically changed for a subsequent iteration of processing input data based the determined accuracy.

Embodiments are at least partly based on the realization that different processing techniques will change in accuracy over time, e.g. as further training data is obtained. Thus, by adjusting a proportion or portion of input data used by each processing technique based on the accuracy of the processing technique, the more accurately that relationships between data items can be identified. It is also recognized that different processing techniques may be better at identifying different relationships, so that it would be advantageous to continually use a plurality of different processing techniques.

Illustrative embodiments may, for example, be employed in the analysis of medical text to identify relationships between entities of the medical text, e.g. between treatment options and outcomes. This improves the analysis of the medical text.

Proposed embodiments provide a means for automating the analysis of input data using two processing techniques. This facilitates using multiple different analysis methods for processing input data.

In the hereafter described embodiments, the "input data" comprises (free-form) textual data, such as a piece or pieces of medical literature. Each iteration of input data may, for example, comprise a difference article or collection of articles about a specific disease domain, such as Hepatocellular Carcinoma (HCC).

The textual data comprises data items, each representing at least one word of the textual data. Data items may, for example, comprise entities described in the textual data, each entity representing a single object or group of objects. An object may, for example, be a noun, optionally including its descriptors.

The detected relationships may be, for example, a semantic relationship (e.g. identical meanings of two words/phrases), or a correspondence relationship (e.g. a concept embodied by a first word/phrase causes a concept embodied by a second word/phrase). Other relationships will be apparent to the skilled person, of which some will be elucidated below.

In other examples, the "input data" may be formed of other data structures, such as an ontology or knowledge graph, datasets of information and so on. Where the input data is an ontology, and the ontology is a tree-based graph structure, a data item may comprise a node of the graph structure.

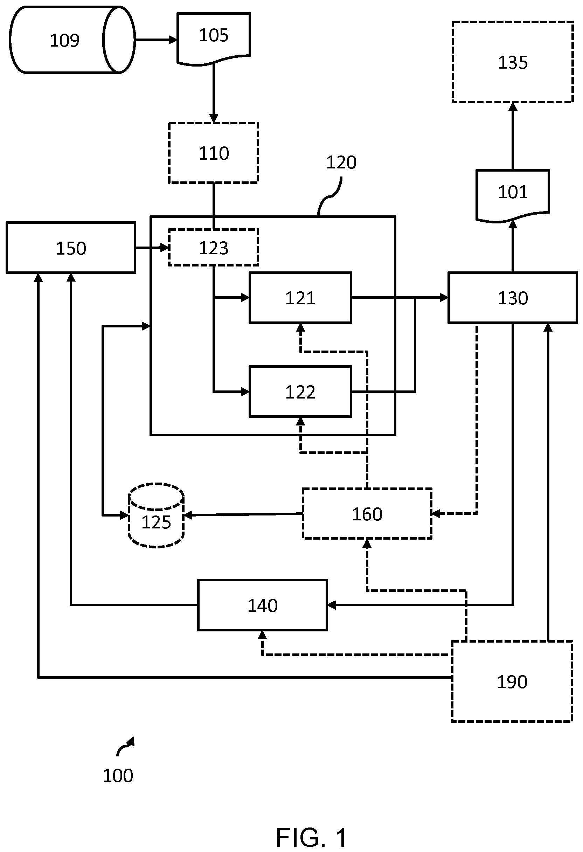

Fig. 1 illustrates the architectural design of a processing system 100 for generating or modifying an ontology 101, representing relationships within input data 105, according to an embodiment of the invention.

The input data 105 may be provided by a data source 109, such as a database like PubMed. An interface (not shown) may enable communications between the processing system 100 and the data source 109.

The processing system 100 comprises a (optional) text data normalizer 110. The normalizer is a component that handles pre-processing of text. The normalizer can carry out simple detection for entities (i.e. specific words or phrases), normalizing them into standardized encoding and then outputting normalized text for more complex analysis. Each detected and standardized entity can act as a data item during subsequent processing.

By way of example, entities detected and normalized at this level can comprise (where the input data 105 comprises an item of medical literature) disease names, chemical names, drug names and gene expressions, for which matured detection tools have already been developed.

Thus, the normalizer may obtain initial input data 105 comprising textual data; process the initial input data using a natural language processing technique to detect entities within the initial input data; and normalize the detected entities into standardized encoding, to thereby generate the input data for further processing.

In some examples, the normalizer may generate an ontology identifying different entities or data items, but without identifying relationships between the entities or data items. The normalizer may be omitted in some embodiments, with the original or initial input data 105 instead being used for further processing rather than the preprocessed input data.

The processing system 100 comprises a relationship detector 120, which comprises at least a first processing module 121 and a second processing module 122.

The first processing module is adapted to process a first portion of the input data using a first processing technique configured to identify relationships between different data items of the input data. The first processing technique may, for example, comprise a rule-based processing technique.

The relationship detector may comprise an input data divider 123 adapted to divide the input data into the first and second portions.

The second processing module 122 is adapted is adapted to process a second, different portion of the input data using a second, different processing technique configured to identify relationships between different data items of the input data. The second processing technique may comprise a machine-learning processing technique.

The first and/or second processing modules 121, 122 may employ cloud computing processing techniques and/or communicate with a memory 125 to perform the first/second processing techniques as would be known to the skilled person. For example, the memory 125 may store reference patterns for a pattern matching technique or a machine learning model for a machine-learning technique.

In some embodiments, the first and/or second processing technique may employ a plurality of subordinate processing techniques to identify relationships between different data items of the input data. Thus, reference to a first processing technique may be replaced by a first set of one or more processing techniques, and reference to a second processing technique may be replaced by a second set of one or more processing techniques.

The size of the first portion is a first percentage of the input data, and the size of the second portion is a second percentage of the input data. In this way, the first and second processing modules process portions of the input data to each identify relationships between data items, e.g. detected entities or different words/phases, of the input data.

By way of example, these relationships may be syntactic relationships (e.g. item A results in item B or item A is caused by item B), semantic/lexical relationships (e.g. item A is equivalent to item B, being an appositive relation, or item A is an example of item B, being a hypernym or hyponym relation), statistical relationships (e.g. item A is greater than item B or item A statistically corresponds to item B) or combination or composite relationship (e.g. item A is used alongside item B).

Each processing technique is adapted to identify relationships between different data items, as will be explained later. Some processing techniques may be better than other processing techniques at identifying certain relationships. By way of example only, the relationship detector may generate a plurality of (semantic) triples, each triple comprising a first data item of the input data, a second data item of the input data and an indication of the relationship between the first and second data items.

In some examples, the first processing technique is a (language) pattern matching technique adapted to identify semantic or grammatical patterns (e.g. identify a subject and object within textual data or identify lists of similar data items) within the text data to derive the relationship between different data items.

Where the first processing technique is a pattern matching technique, the relationship detector 120 may communicate with a memory 125 that stores reference (language) patterns and a relationship corresponding to the reference pattern. The pattern matching technique may compare the reference patterns to the input data to identify semantically or syntactically similar patterns, and associate the similar patterns with the relevant relationship of the reference pattern.

In examples, the second processing technique is a machine-learning processing technique, e.g. using a neural network. A machine-learning technique may be adapted to identify relationships between underlying concepts represented by data items, as would be known by the skilled person. Examples of suitable machine-learning processing techniques will be elucidated later in the description.

The first processing technique may be a shallow matching technique, i.e. only capable of utilizing textual level pattern matching (e.g. identifying semantic or grammatical patterns), and the second processing technique may be a deep matching technique, e.g. utilizing knowledge level similarity.

The processing system 100 further comprises an ontology generating module 130 adapted to generate or modify an ontology based on the relationships between the different data items identified by the first and second processing techniques. Thus, the ontology generating module 130 combines or synthesizes the relationships identified by the first and second processing modules 121, 122 of the relationship detector 120 to generate or modify an ontology.

It is pointed out that methods of generating or structuring an ontology are well known in the prior art. Examples of suitable ontologies include a knowledge graph, other graph structures (storable in a "graph database", e.g. which employs a property graph model) or a dataset of (semantic) triples.

By way of example, the ontology generating module may construct a knowledge graph that identifies different data items (or entities) and the relationships between them. A knowledge graph is a tree-based structure in which each node represents a different data item or entity, and the branches between the nodes representing the relationship between the connected data items. A full example of a knowledge graph will be provided later.

In one example, where the processing system 100 comprises a text data normalizer 110, the ontology-generating module 130 is adapted to populate a knowledge graph with entities detected by the text data normalizer. The ontology-generating module may then define relationships between the different entities based on the detected relationships.

Thus, at least initially, a knowledge graph may contain only source data information (e.g. articles, sentences and tokens) and no extracted relations (which are later inserted). To embed relationships within this knowledge graph, it can be extended by 'growing' new branches to include said information in the knowledge graph.

In another example, where the relationship generator generates a plurality of (semantic) triples, the ontology-generating module may populate a knowledge graph with different data items from the plurality of triples (e.g. identifying unique data items) and subsequently defining relationships between the populated data items of the knowledge graph.

In yet another example, where the relationship generator generates a plurality of (semantic) triples, the ontology-generating module may generate a triple dataset from the plurality of semantic triples. Generating the triple dataset may comprise further processing the plurality of semantic triples, e.g. by discarding duplicate triples or organizing the triples so that those containing similar data items are proximate to one another.

In embodiments, the processing system 100 may comprise a display device 135 adapted to display the generated or modified ontology to a user. This enables the user to intuitively identify relationships between data items of input data, avoiding the need for the user to read through or investigate the entirety of the input data.

The processing system 100 further comprises an accuracy-determining module 140 adapted to determine an accuracy of each of the first and second processing techniques. The accuracy-determining module 140 thereby determines how accurate the first and second processing techniques are at identifying relationships between data items of the input data.

Various embodiments of the accuracy-determining module 140 are envisaged by the present invention.

In a first example, the accuracy-determining module may obtain validation input data, comprising a plurality of validation data items, and validation answer data indicating relationships between the validation data items of the validation input data. The accuracy determining module may process a first instance (or portion) of the validation input data using the first processing technique and a second instance (or portion) of the validation data using the second processing technique to generate first and second validation output data respectively. An accuracy of each processing technique can then be established by comparing the first/second validation output data to the validation answer data.

Methods of determining an accuracy using validation information (input and answer data) will be well known to the skilled person, and may include determining a sensitivity, specificity and/or error function of a processing technique using the validation information (any of which may act as a measure of accuracy for the purpose of the present invention).

This validation answer data may be provided by a user, e.g. working alongside the automated processing of the input data. In some examples, the validation answer data comprises a user-corrected version of the ontology produced by the ontology-generating module.

The validation input data and validation answer data effectively provide ground truth information or dataset for establishing an accuracy of the first/second processing techniques. The ground truth dataset may, for example, be data established/published by experts in the target area (e.g. disease domain), which can be imported into the processing system (e.g. into the memory 125) and used as a reference by the accuracy-determining module 140.

In a second example, a user may be able to correct the ontology generated by the ontology generating module 130 via one or more user correction signals. Each user correction signals indicates a user-identified change or correction to a relationship (or data item) of the ontology. The accuracy determining module 140 may receive these user correction signals to determine an accuracy of the first/second processing technique. Effectively, this enables the accuracy of the first/second processing techniques to be determined based on manual corrections of the user (i.e. manually).

In particular, a user correction signal indicates that the first/second processing technique (that identified the incorrect relationship or data item) was inaccurate. The greater the number of user correction techniques, the less accurate the corresponding processing technique. The accuracy may be represented as a simple count of the number of user corrections signals associated with a particular processing technique, or as a percentage of the number of relationships predicted by the said processing technique that were corrected (e.g. 30% of predictions needed correction). This indicates the accuracy of the processing technique.

The user correction signal may be provided from a user interface 190, effectively acting as a knowledge editor. Thus, the user may view the generated ontology (e.g. on the display 135), and generate one or more user correction signals for correcting the generated ontology. These user correction signals may be used to define the accuracy of the first/second processing techniques and/or to retrain the first/second processing techniques (as set out below).

The processing system 100 further comprises a size adjustment module 150 that adjusts a size of the first and second percentages, for processing of future input data, based on the determined accuracy of each of the first and second processing techniques. In other words, the weighting of the first and second processing techniques may depend upon the accuracy of the first and second techniques.

The size adjustment module 150 maybe adapted to communicate with the input data divider 123 to control the size of the portions for future input data.

In a first example, if the accuracy determining module 140 determines that the first processing technique was more accurate (e.g. by more than a predetermined accuracy value) than the second processing technique, then the size of the first percentage may be increased, and the size of the second percentage decreased (and vice versa). The size of the change to the first/second percentage maybe predetermined (e.g. a 5% or 10% change), based on a difference between the two percentages (e.g. reduce a difference by 10%) or based upon the difference between the accuracies. A predetermined size/percentage may be defined by a user, e.g. via a user interface.

In a second example, the size adjustment module 150 maybe adapted to decrease the size of the first/second percentage in response to a user correction signal indicating that the first/second processing technique was incorrect or inaccurate. The accuracy-determining module may be adapted to pass this information to the size adjustment module.

Preferably, the size adjustment module is adapted to not reduce the size of either of the first or second percentages below a predetermined value (e.g. below 5%).

Other examples will be apparent to the skilled person.

In this way, the size of the first and second percentages (representing the proportion of the input data processed by the first and second processing techniques respectively) is adaptive and responsive to the (in)accuracy of the first and second processing techniques. Thus, the more errors in a processing technique, the less that the said processing technique is relied upon to determine the relationship between different data items of the input data.

Over time, e.g. as the processing techniques are adapted or trained, the accuracy of the processing technique(s) may improve or change. Thus, the more accurate processing techniques may automatically be used to identify relationships within the input data. Thus, a balance or compromise is automatically performed between the two processing techniques by the size adjustment module 150 changing the value of the first and second percentages based on an accuracy of the corresponding processing techniques.

The initial values of the first and second percentages, i.e. during a first iteration, maybe preset.

In preferable embodiments, for the first iteration, the size of the first percentage is greater than the size of the second percentage. This is particularly advantageous when the first processing technique is a rules-based (e.g. pattern matching) technique and the second processing technique is a machine-learning technique, as rules-based techniques are generally considered to be more reliable or accurate with less training than machine-learning techniques.

In particular examples, for the first iteration, the size of the first percentage is between 80% and 95% (e.g. 95%) and the size of the second percentage is between 5% and 20% (e.g. 5%), wherein the total of the first and second percentages is no greater than 100%.

In some further embodiments, the size adjustment module 150 maybe adapted to determine whether a manual override signal provides information on a user's desired size of the first and/or second percentage. In response to the manual override signal providing information on a user's desired size, the size adjustment signal may adjust the size of the first and/or second percentage based on the manual override signal.

Thus, the user maybe able to override the automatic adjustment to the first and second percentages using a manual override signal. The manual override signal may be provided via a user interface 190.

Optionally, the processing system 100 may further comprise a training module 160 adapted to retrain or further train at least one of the first and second processing techniques using training input data and training answer data (i.e. ground truth information). This may comprise updating a memory 125 storing information sued by the first and/or second processing modules to perform the first/second processing techniques.

The training module 160 maybe adapted to (re)train the first/second processing techniques before an accuracy is determined by the accuracy determining module 140.

The training input data may correspond to the input data initially processed by the processing system, and the training answer data may correspond to the ontology generated by the ontology-generating module, after it has been corrected by a user. Thus, a user-corrected ontology maybe used to derive at least some of the training answer data (as the user-corrected ontology will provide an indication of relationships between data items of the corresponding training input data).

As previously set out, a user may view a generated ontology (e.g. on the display 135), and generate one or more user correction signals for correcting the generated ontology via a user interface 190. The corrected ontology, and its associated input data, can then be provided as additional training data (i.e. new ground truth information) and used to retrain the first/second processing technique.

In embodiments, at least some of the training input data and training answer data is provided by an external training dataset, which may be periodically updated (e.g. as new studies or information is made available, or as other instances of the claimed method is performed). Thus, in some embodiments the training input data and training answer data is provided from a combination of existing training data and information generated by performing the method.

In some embodiments, the results of the claimed method are stored on a dataset for other instances of the claimed method to perform training.

(Re)training may be performed after every iteration of generating/modifying an ontology, or after a certain number of iterations has been performed (to save on processing power and improve an amount of training data available). For example, the first/second processing techniques may be retrained only after at least 5 iterations or at least 10 iterations or at least 100 iterations or at least 300 iterations of processing input data to generate/modify the ontology have been performed. The number of iterations may be defined by the user, e.g. via the user interface 190.

In other examples, (re)training may be performed after a certain period of (accumulated) time of performing the iterations, e.g. after 5 hours of performing the iterations or after 1 hour of performing the iterations. The certain period of time maybe defined by the user, e.g. via the user interface 190.

Where more than one iteration is performed before retraining, a plurality of user-corrected ontologies may be made available for (re)training the first/second processing technique.

In some embodiments, a user may be able to submit new knowledge via a user interface 190, which can thereby act as a knowledge editor, e.g. identify new relationships between different data items. This new knowledge may be integrated into the generated ontology, e.g. by the ontology generating module 130.

Thus, the ontology-generating module may determine whether a user input signal provides information on relationships between different data items of the input data; and in response to the user input signal providing information on relationships, generate or modify the ontology further based on the user input signal.

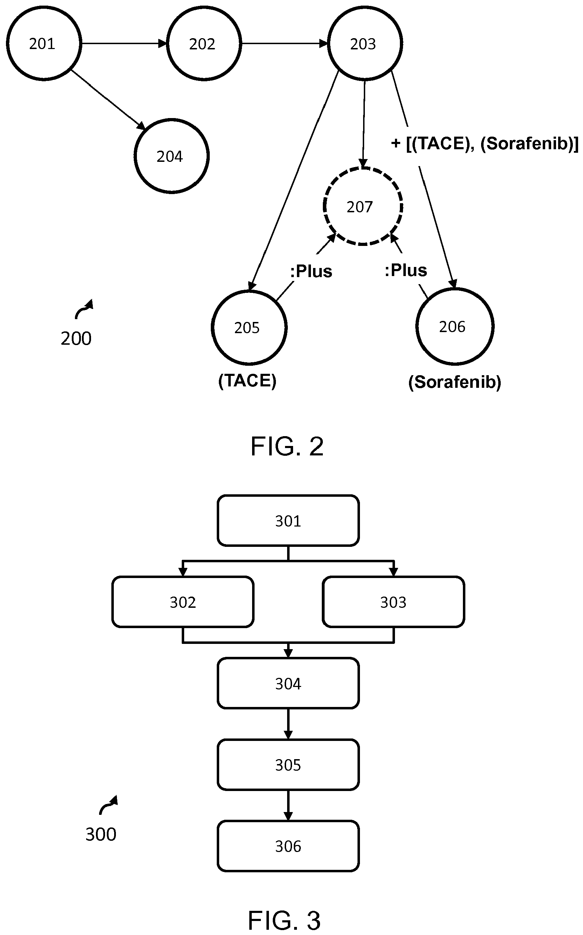

An example of a suitable ontology, to be generated/modified by the ontology-generating module 130, will hereafter be described with reference to Fig. 2. In particular, Fig. 2 conceptually illustrates the structure of a tree-based ontology 200.

In the example ontology, special notations are used to encode or define the relationships between two data items (or entities). An equal (=) notation can represent an appositive relation (i.e. semantically identical relationship) between two data items. A greater-than sign (>) notation and a less-than sign (<) can represent hypernym and hyponym relationships respectively. Finally, plus (+) notation can represent a composite relation.

By way of example, consider an extract of input text stating that:
"The purpose of our study was to test the hypothesis that sorafenib-related dermatologic adverse events (AEs) as an early biomarker can predict the long-term outcomes following the combination therapy of transarterial chemoembolization (TACE) plus sorafenib (TACE-S)".

A (first or second) processing technique may be able to identify that the phrase "combinational therapy of transarterial chemoembolization (TACE) plus sorafenib" indicates an appositive relation between TACE and sorafenib, e.g. by recognizing a semantic pattern with the word "plus". It is therefore possible to create an ontological indication indicating that TACE and sorafenib can be combined, e.g. using the notation +(TACE,Sorafenib).

As a further example, input data may describe how PRFA is a kind of treatment belonging to the family of palliative treatment, where the hypernym is captured by the notation of <(PRFA,Palliative Treatment). Similarly, it is possible to represent appositive relationships of three different treatment modalities with the formula: =(TACE,Microwave Ablation,RFA). By using a combination of this set of notations, more complex relationships between data items or entities can be represented in formulaic form, e.g. =(TACE,+(TACE,Sorafenib)). This formula represents an appositive relation between TACE and the composition of TACE and Sorafenib.

The ontology may be formed in a tree structure, which can be encoded as node and relationships inside a knowledge graph. For example, a predicate node can be used to represent a relation type (e.g. notation) between two data items or entity, with an entity node being used to store an entity. These nodes can be appropriately connected together to encode the desired relationship.

These relationships may be called language patterns and can be extracted from the narrative data of medical literatures or existing ontologies, e.g. by employing methods according to embodiments of the invention.

Fig. 2 illustrates an ontology 200 or knowledge graph in which the relationship between two data items has been inserted.

The ontology is a tree-based structure, with different entity nodes 201 - 206 representing different data items or entities of the input data. In particular, the ontology may be a "graph structure", which is a known data structure for storing data items (commonly labelled "nodes" or "entities") and the relationship between said data items.

By this design, it is possible to capture the context of detected knowledge at multiple granularity level in terms of word tokens, sentences and paragraphs. Starting at sentence level, we can follow the links in the graph to retrieve corresponding, paragraph and article level by level. This can be achieved via common graph query languages.

The entity nodes may, for example, be created by the ontology-generating module based on normalized text. In particular, the text data normalizer may identify different data items (or "entities") within input text, e.g. labels of drugs or treatments, and the ontology-generating module may populate or create entity nodes 201-206 with the identified data items.

In one example, where the processing system 100 comprises a text data normalizer 110, the ontology-generating module 130 is adapted to populate a knowledge graph with entities detected by the text data normalizer. The ontology-generating module may then define relationships between the different entities based on the detected relationships.

At least one additional node 207 (e.g. predicate nodes) may then be generated to define a respective at least one relationship between different entities represented by the entity nodes 201-206. The content of the additional node 207 may indicate the type of relationship, e.g. apposition relation, hypernym/hyponym relation or composite relation.

By way of example only, the relation may defined using a JavaScript Object Notation (JSON) format. It is possible to create or define a standardized JSON structure for user to specify knowledge, i.e. entities and relations inside a textual context. Multiple modalities (i.e. predicate nodes or additional nodes) can be placed inside the array structure. Each modality or additional node could define name, alias, pattern expression, relationship type (+,<,>,()), and sub-components which are recursively defined structures. Type notation () represents entity type at leaf node, which contains no more sub-components.

One example of an additional node 207 maybe represented as:

```
 {
 "id": XXXX,
 "name": "sorafenib transcatheter arterial chemoembolization",
 "type": "+",
 "expression": "+[(TACE), (Sorafenib)]",
 "category": "Modality",
 "components": [...]
 }
```

Thus, a PLUS relation 207 between two entities can be stored in JSON format identifying two child nodes 205, 206 (TACE and Sorafenib). Child nodes may be defined recursively.

For the sake of improved clarity, Fig. 2 provides text illustrating this specific example.

It has previously been described how two different processing techniques may be used to process the input data. The first processing technique may be a shallow matching technique, and the second processing technique may be a deep matching technique. Shallow matching techniques can employ a rule-based processing technique to identify predetermined patterns within input data. Deep matching techniques can employ a machine-learning processing technique to identify less well-defined relations between data items in input data and beyond.

A shallow matching technique may use reference language patterns, including syntactic (e.g. grammatical) patterns and semantic patterns, to process the corresponding portion of the input data. Thus, the shallow matching technique may effectively be a language pattern matching technique. Each reference language pattern can be associated with a particular relation type (e.g. =, <, > or +), and may comprise a sample sentence (i.e. define sample data items and their relationship). The shallow matching technique may retrieve sentences with similar language patterns to reference language patterns from the input data, and associate the retrieved sentences with a same relation type (<, >, + or =) as the corresponding reference language pattern, thereby effectively associating a relationship between entities within the retrieved sentence. This can be achieved by applying standard/existing syntactic and semantic parsers. It is not necessary for the entities (i.e. data items) included in the retrieved sentences to be same as in the reference language patterns. In this phase, the shallow matching technique merely 'guesses' relationships between data items by suggesting all sentences with similar patterns.

In other words, a shallow matching technique may identify language patterns within the input data that are similar to known or reference language patterns (associated with a certain relationship) to identify data items and their relationship.

By contrast, a deep matching technique aims to find knowledge level similarity (rather than mere surface pattern similarity). A relationship between data items is connected to its context in the input data. A deep matching algorithm is able to trace certain data items back to its context to find other concepts or data items in this context and link to detected concepts (i.e. establish knowledge-based relationships between data items).

In further examples, the input data may be connected with or link to other data sources (e.g. via referencing or footnotes in medical literature). The deep matching technique maybe capable of further crawling to identify linked concepts in the other data sources.

The result is a graph of linked concepts that surround the detected data items. It is possible to encode such networked relations into vectorized representation via graph embedding techniques. The vectorized representation can then be utilized to compute similarities between detected concepts and other concepts in the corpus. Sentences with high similarity concepts can be identified and associated with one another, i.e. a relationship can be identified. In this way, the deep matching technique may employ concept or knowledge level matching to identify matching, or the relations between, underlying concepts of data items within the input data.

In any described embodiment, the complexity of the first/second processing technique may be modifiable. By way of example only, if the first processing technique is a rules-based technique, the number or complexity of rules used may be modifiable. In particular, if the first processing technique utilized a pattern matching methodology, the number or complexity of reference patterns may be modified.

In at least one embodiment, the complexity of the first and second technique may be modified based upon the accuracy of the first/second technique. Thus, the processing system may further comprise a complexity modifier adapted to modify the complexity of the first/second technique based on the respective accuracy of the first/second technique. In particular, the complexity of the first/second technique may be increased if the accuracy of that technique is greater than a first predetermined value, and decreased if below a second predetermined value. The first and second predetermined values may be the same or (preferably) different.

In other embodiments, the complexity of the first/second technique may be modified based on a user input, e.g. received by the user interface. This allows this user to control the complexity of the first/second processing technique.

Fig. 3 illustrates a method 300 according to an embodiment of the invention. The method 300 is iteratively repeated.

The method 300 comprises a first step 301 of obtaining input data for processing, the input data comprising a plurality of data items.

The method 300 further comprises a second step 302 of processing a first portion of the input data using a first processing technique configured to identify relationships between different data items of the input data, the size of the first portion being a first percentage of the input data; and a third step 303 of processing a second, different portion of the input data using a second, different processing technique configured to identify relationships between different data items of the input data, the size of the second portion being a second percentage of the input data. The second 302 and third 303 steps maybe performed concurrently.

The method 300 further comprises a fourth step 304 generating or modifying an ontology based on the relationships between the different data items identified by the first and second processing techniques. The method 300 comprises a fifth step 305 of determining an accuracy of each of the first and second processing techniques.

The method 300 further comprises a sixth step 306 of adjusting a size of the first and second percentages, for processing of future input data, based on the determined accuracy of each of the first and second processing techniques.

Embodiments have been described with reference to only two processing techniques, but the methodology may be expanded to operate with three or more processing techniques, by adapting the procedure previously described. For example, there may be first, second and third percentages which are iteratively modified. By way of example, a third processing technique may be included that integrates rule-based and learning based techniques.

A machine-learning algorithm is any self-training algorithm that processes input data in order to produce or predict output data. Here, the input data comprises data items and the output data comprises relationships between the data items of the input data.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian model are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example data items of input data. The training output data entries correspond to relationships between the example data items, which may be derived from a user-corrected ontology.

Use of the knowledge graph structure improves entity and relationship detection accuracy compared to other methods of modelling the relationships between entities.

The knowledge graph structure links entities relative to its context, which here refers to the sentences, paragraphs and/or articles forming the input data. An article usually contains some meta information (such as title, keywords, topic, category, authors, institutions and so on), which can act as contextual information that can be exploited to improve relationship detection accuracy. In particular, context information can serve as rich features for improving a relationship detection algorithm (e.g. machine learning process) and accelerate the iterative process. With the graph-based structure, this should be carried out more easily, as such contextual information may be automatically included within the graph-based structure.

Consider a scenario in which, at least initially, there is limited training data for training a processing technique for identifying relationships. The user may provide some training data, e.g. by annotating some input data using a knowledge editor. In this example, the user annotates the input data to identify a first entity ("TACE"), a second entity ("Sorafenib") and a relationship ("PLUS" or "+"). Because this annotation is associated with a specific sentence of a specific article, some contextual information surrounding the relationship can be extracted and serve as a basis retrieving and ranking more articles and sentences which may include the same PLUS relation so that can be recommended to user to verify.

For example, the contextual information could be the sentences that explain such relations in the user-annotated article. These sentences can then be turned into vector representations (e.g. using doc2vec). It is possible to further take more features into representation, such as using the title and keywords of an article and turn them into vector representation. Moreover, it is also possible to leverage the path information inside the graph (from sentence node up to article node), to represent a path in the graph into vectors. There are already matured algorithm for performing this process (e.g. TransE, TransH).

With the above feature representations, it is possible to do several things, examples of which are listed below:
1) Retrieval and ranking of other sentences, paragraphs or articles, which are similar at a semantic level to current (i.e. recently annotated) ones. Using a knowledge graph structure, this selection is relatively straightforward, by selecting a group of sentences or even paths. Candidates that may include the same relation can be identified as presented to the user for checking (i.e. to provide further training examples);
2) Retrieval and ranking of other sentences, paragraphs or articles, which are the most dissimilar, to provide more diversified samples for user to annotate. This falls into the category of active learning, which is aimed at accelerating annotation process with the least training samples;
3) During training, the extended features abovementioned can contribute directly to the training process so that the model is more context-aware. With growing contexts associated to the pattern under the structure, the training can leverage more narrative and path features to improve the self-learning process.

Furthermore, during training, in a deep learning context, it is possible to use the entire graph structure as a feature (e.g. an element of the input data that may influence the output data - which here is the identified entities and relationship(s)). As is conventional with machine-learning algorithms, as the algorithm is trained, it will self-select which are important features and relevant features.

That being said, in some scenarios it may be preferred to allow the user to add some preference for certain features (i.e. the user may control or influence which elements of the input data have an influence on output data). Thus, some embodiments may allow the user to define or control which aspects of the input data have an influence on the output data of the machine-learning process. The user therefore has the flexibility to decide which features or paths to use. This may be controlled via the user interface.

For instance, for path feature, a user can put complex query to select, e.g. for training, those articles co-authored by two authors (who are famous for research regarding TACE), under a specific research topic, and with the frequency of specified keywords beyond a threshold. Graph structure is especially good at handling such query. In this way, the system becomes more focused on learning and detecting TACE related entities and relations.

The skilled person would be readily capable of developing a processing system for carrying out any herein described method and vice versa. Thus, each block of a diagram may represent a different step performed by a method or a different action performed by a processing system, which may be performed by a respective module of the processing system.

Embodiments may therefore make use of a processing system. The processing system can be implemented in numerous ways, with software and/or hardware, to perform the various functions required. A processor is one example of a processing system that employs one or more microprocessors that may be programmed using software (e.g., microcode) to perform the required functions. A processing system may however be implemented with or without employing a processor, and maybe implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs). In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved.

Embodiments have been described with reference to input data formed of text in the English language, but it is envisaged that embodiments may expand to input data having text in any known natural language, as well as non-textual data.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A computer-implemented method of generating or modifying an ontology representing relationships within input data, the method comprising iteratively:
- obtaining input data for processing, the input data comprising a plurality of data items;
- processing a first portion of the input data using a first processing technique configured to identify relationships between different data items of the input data, the size of the first portion being a first percentage of the input data;
- processing a second, different portion of the input data using a second, different processing technique configured to identify relationships between different data items of the input data, the size of the second portion being a second percentage of the input data;
- generating or modifying an ontology based on the relationships between the different data items identified by the first and second processing techniques;
- determining an accuracy of each of the first and second processing techniques; and
- adjusting a size of the first and second percentages, for processing of future input data, based on the determined accuracy of each of the first and second processing techniques.

2. The computer-implemented method of claim 1, wherein the input data for processing comprises textual data, each data item representing at least one word of the textual data.

3. The computer-implemented method of claim 2, wherein the step of obtaining input data comprises:
- obtaining initial input data comprising textual data;
- processing the initial input data using a natural language processing technique to detect entities within the initial input data; and
- normalizing the detected entities into standardized encoding, to thereby generate the input data for further processing.

4. The computer-implemented method of any of claims 1 to 3, wherein the first processing technique comprises a rule-based processing technique and the second processing technique comprises a machine-learning processing technique, such as a support-vector machine processing technique or a neural network processing technique.

5. The computer-implemented method of claim 4, wherein, for the first iteration, the size of the first percentage is greater than the size of the second percentage.

6. The computer-implemented method of claim 5, wherein, for the first iteration, the size of the first percentage is between 80% and 95% and the size of the second percentage is between 5% and 20%, wherein the total of the first and second percentages is no greater than 100%.

7. The computer-implemented method of any of claims 1 to 6, wherein the step of determining an accuracy of each of the first and second processing techniques comprises:
- obtaining validation input data comprising a plurality of validation data items;
- obtaining validation answer data indicating relationships between the validation data items of the validation input data;
- processing validation input data using the first processing technique to generate first validation output data predicting relationships between different validation data items of the validation input data; -
- comparing the first validation output data to the validation answer data to determine an accuracy of the first processing technique;
- processing the validation input data using the second processing technique to generate second validation output data predicting relationships between different validation data items of the validation input data; and
- comparing the second validation output data to the validation answer data to determine an accuracy of the second processing technique.

8. The computer-implemented method of any of claims 1 to 6, wherein the step of determining an accuracy of each of the first and second processing techniques comprises:
- receiving one or more user correction signals, each user correction signal indicating a user-identified correction or change to a relationship between the different data items identified by the first and second processing techniques; and
- determining an accuracy of the first and second processing techniques based on the user correction signals.

9. The computer-implemented method of any of claims 1 to 8, further comprising, between iterations, retraining or further training at least one of the first and second processing techniques using training input data and training answer data.

10. The computer-implemented method of any of claims 1 to 9, wherein the ontology is a tree-based structure comprising nodes, each node representing a different data item, and connections between nodes, each connection representing a relationship between data items represented by the nodes, wherein the ontology is optionally a graph structure.

11. The computer-implemented method of claim 10, wherein the adjusting a size of the first and second percentages comprises:
- determining whether a manual override signal provides information on a user's desired size of the first and/or second percentage; and
- in response to the manual override signal providing information on a user's desired size, adjusting the size of the first and/or second percentage based on the manual override signal.

12. The computer-implemented method of any of claims 1 to 11, wherein the step of generating or modifying an ontology further comprises:
- determining whether a user input signal provides information on relationships between different data items of the input data; and
- in response to the user input signal providing information on relationships, generating or modifying the ontology further based on the user input signal.

13. The computer-implemented method of any of claims 1 to 12, further comprising a step of processing a third portion, different to the first and second portions of the input data using a third, different processing technique configured to identify relationships between different data items of the input data, the size of the third portion being a third percentage of the input data,
wherein:
the step of generating or modifying an ontology is further based on the relationships identified by the third processing technique;
the step of determining an accuracy further comprises determining an accuracy of the third processing technique;
and the step of adjusting a size further comprises adjusting a size of the third percentage based on the determined accuracy of at least the third processing technique.

14. A computer program comprising code means for implementing the method of any one of claims 1 to 13 when said program is run on a processing system.

15. A processing system for generating or modifying an ontology representing relationships within input data, the processing system comprising:
an input module adapted to obtain input data for processing, the input data comprising a plurality of data items;
- a relationship detector comprising:
- a first processing module adapted to process a first portion of the input data using a first processing technique configured to identify relationships between different data items of the input data, the size of the first portion being a first percentage of the input data,
- a second processing module adapted to process a second, different portion of the input data using a second, different processing technique configured to identify relationships between different data items of the input data, the size of the second portion being a second percentage of the input data,
- an ontology generating module adapted to generate or modify an ontology based on the relationships between the different data items identified by the first and second processing techniques;
- an accuracy determining module adapted to determine an accuracy of each of the first and second processing techniques; and
- a size adjustment module adapted to adjust a size of the first and second percentages, for processing of future input data, based on the determined accuracy of each of the first and second processing techniques.
